(19) **Europäisches Patentamt / European Patent Office / Office européen des brevets**

(11) **EP 4 282 366 A1**

(12) **EUROPEAN PATENT APPLICATION**

(43) Date of publication:
**29.11.2023 Bulletin 2023/48**

(21) Application number: **22174889.0**

(22) Date of filing: **23.05.2022**

(51) International Patent Classification (IPC):
**A61B 34/20** (2016.01)

(52) Cooperative Patent Classification (CPC):
**G02B 6/26; G02B 6/02042;** A61B 2034/2061

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **POTZE, Willem**
  **Eindhoven (NL)**
- **HARDEMAN, Toon**
  **Eindhoven (NL)**
- **LEISTIKOW, Merel Daniëlle**
  **5656AG Eindhoven (NL)**
- **VAN PUTTEN, Eibert Gerjan**
  **5656AG Eindhoven (NL)**
- **DENISSEN, Sander Hans**
  **Eindhoven (NL)**

(74) Representative: **Philips Intellectual Property & Standards**
**High Tech Campus 52**
**5656 AG Eindhoven (NL)**

(54) **SHAPE REGISTRATION OF A SHAPE-SENSING ENABLED DEVICE TO AN IMAGING SYSTEM**

(57)     A method of registering part of an optical shape sensing system (10) to an imaging system (48) is described, said part comprising a distal multicore optical fiber (16) and a proximal multicore optical fiber (14), an optical connector (34) optically connecting the distal optical fiber (16) and the proximal optical fiber (14) to each other, a launch base (36) fixed with respect to the imaging system (48) and arranged to fix the proximal optical fiber (14). In the method, the shape and orientation of the distal optical fiber (16) and of a section of the proximal fiber (14) extending from the optical connector (34) to the launch base (36) are optically measured for at least two different positions and/or orientations of the optical connector (34) with respect to the object (28). Inter alia based on the optical measurements in the first and second positions or and/orientations of the optical connector (34), said part of the optical shape system as a whole is registered to the imaging system (48). A system for carrying out the method is also described.

Fig. 7

EP 4 282 366 A1

**Description**

FIELD OF THE INVENTION

The present invention generally relates to the field of optical shape sensing (OSS) using optical fiber sensors, also referred to as FORS (Fiber Optic RealShape). In particular, the present invention relates to a method of registering part of an optical shape sensing system to an imaging system, for example an X-ray or MR (magnetic resonance) imaging system. More particularly, the present invention relates to a method of registering part of an optical shape sensing system to an imaging system, wherein said part comprises a distal multicore optical fiber optically connected to a proximal multicore optical fiber through an optical connector which has back loading capability. The invention further relates to a corresponding system and a computer program.

BACKGROUND OF THE INVENTION

While the present description refers to the use of FORS in the medical or surgical field, it is to be understood that the invention is not limited thereto.
In the medical field, there is a clear and ongoing trend to replace conventional surgical procedures with minimally invasive interventions. In these minimally invasive interventions, medical instruments, such as guide wires and catheters, are inserted into the body through small incisions. Several visualization techniques exist to navigate a medical instrument to the required location inside the body.
FORS is a technology to track a device, e.g a guide wire equipped with an optical fiber, in the body of a patient. The whole three-dimensional shape of the device can be reconstructed from the optical interrogation of the optical fiber comprised by the device. The exact orientation and position of the device is determined in real-time in an appropriate coordinate system. In FORS, geometrical changes of the device are encoded into the light field that propagates through the optical fiber integrated in the device. Optical interrogation of this optical fiber gives the information needed to, in principle, reconstruct the three-dimensional shape of the whole optical fiber (and hence that of the device), in real time. The reconstructed shape of the optical fiber (hence of the medical device into which the fiber is integrated) is typically displayed in a relevant coordinate system, such as one that matches an operating theatre in which FORS is used, for example a coordinate system which is linked to an imaging system, e.g. an X-ray imaging system or an MR imaging system. The shape and orientation of the FORS enabled device can then be visualized co-aligned with an image of the object, e.g. a vessel structure of the patient, which is provided by the imaging system beforehand or during navigation of the device. To this end, the FORS enabled device is typically registered to the relevant coordinate system, for example during setup using, e.g., one or more X-ray or MR images.
The optical fiber that is being used to determine the shape of the device (FORS sensor) typically comprises multiple optical cores, for example a central core and multiple outer cores that may spiral around the central core along the length of the optical fiber. For example, an optical fiber may comprise four cores, one central core and three outer cores that may be located at nominally 120° from each other at a fixed distance from the central core.
To make a functional connection between a distal optical fiber, for example an optical fiber integrated into a medical device, like a guide wire, and a proximal optical fiber, for example an optical fiber integrated into a patch cord connected to the optical interrogator, it is important to align the fiber cores of the distal fiber with the cores of the proximal fiber. A common way to achieve such an alignment is to assemble both fibers into an optical connector and mate them in a mating sleeve. Low tolerance elements in the connector, such as ceramic ferrules, ensure the centering of the two fibers inside the mating sleeve. A connector key defines the angular alignment between the two optical fibers.
For certain designs of medical devices, for example guide wires, in particular back loadable guide wires, where the outer diameter typically cannot be larger than a fraction of a millimeter, a robust connector key is difficult to manufacture. In some surgical procedures, the catheter around the optical guide wire needs to be replaced by another one to carry out a medical treatment of the patient. In those procedures, the optical guide wire stays inside the patient, while the catheter around the guide wire is replaced by another one. A back loadable optical connector is needed, which can disconnect the distal optical fiber (e.g. of the guide wire) from the proximal optical fiber (e.g. of the patch cord), to interchange the catheter. After exchanging the catheter, the distal and proximal optical fibers are reconnected in the optical connector.
In certain foreseen FORS system setups, especially setups that support back loadable guidewires, both the shape of the back loadable guidewire and the shape of part of the patch cord between the interrogator and the guidewire are reconstructed. These two shapes are combined at the point of the optical connection. Due to manufacturing tolerances and misalignments in the connector, the exact relative orientation between the two shapes might not be fully known, thereby having a detrimental effect on the accuracy of the predicted position and orientation of the parts of the medical device (e.g. guide wire) inside the patient.
Thus, for a proper functioning of the optical back loadable connector, it is important that the central and outer cores in the part of the optical fiber that is still partly inside the patient are properly aligned with those in the part of the fiber that is connected to the interrogator. This holds for an initial connection of the two fibers as well as for a reconnection of the two fibers after a disconnection. A method must be found and implemented to compensate for a possible improper alignment of the two parts of the distal and proximal fibers in the connector. Due to non-zero tolerances in the fabrication process of the guide wire including an optical fiber and in that of the optical connector, it is most likely that a misalignment between the distal and the proximal optical fiber exists. These non-zero tolerances, and hence misalignments, have a detrimental effect on the accuracy of the predicted position and orientation of the parts of the medical device (e.g. guide wire) inside the patient, especially near its distal tip. Accurate knowledge of the position of the distal tip of the medical device is essential for proper treatments of patients.

Hence, there still is a need in a method and system that allows for highly accurate determination of position and orientation of the distal optical fiber, e.g. comprised by an optical guide wire, in the relevant coordinate system of an imaging system, despite of non-zero tolerances in the manufacturing process of the optical fibers and of the optical connector.

SUMMARY OF THE INVENTION

It is an object of the invention to provide a method of registering an optical shape sensing system comprising a distal multicore optical fiber, a proximal multicore optical fiber and an optical connector therebetween, to an imaging system, which compensates for a possible improper alignment of the distal and the proximal optical fiber in the connector.

It is a further object of the present invention to provide a method of registering an optical shape sensing system to an imaging system which allows an accurate re-registration after a disconnect and reconnect between the proximal optical fiber and the distal optical fiber.

It is a further object of the present invention to provide an optical shape sensing system which makes use of the method according to the invention.

It is a still further object of the present invention to provide a computer program to carry out the method according to the invention.

According to a first aspect of the invention, a method of registering part of an optical shape sensing system to an imaging system is provided, said part comprising a distal multicore optical fiber and a proximal multicore optical fiber, an optical connector optically connecting the distal optical fiber and the proximal optical fiber to each other, a launch base fixed with respect to the imaging system and arranged to fix the proximal optical fiber, the method comprising:

i) providing data of at least a portion of the distal optical fiber from images of the imaging system;
ii) from the positioning of the optical connector in a first position and/or orientation, and in the first position or orientation of the optical connector:

- optically measuring the shape and orientation of the distal optical fiber,
- optically measuring the shape and orientation of a section of the proximal fiber extending from the optical connector to the launch base,

iii) from the positioning of the optical connector in a second position and/or orientation different from the first position or orientation, and in the second position and/or orientation of the optical connector:

- optically measuring the shape and orientation of the distal optical fiber,
- optically measuring the shape and orientation of said section of the proximal fiber,

iv) registering at least said portion of the distal fiber to the imaging system based on a shape-to-shape basis between at least one of the optical shape measurements in the first and second positions and/or orientations of the optical connector and the provided data,
v) based on the optical measurements in the first and second positions and/or orientations of the optical connector, registering said part of the optical shape system as a whole, including at the optical connector and at the launch base, to the imaging system.

The method according to the invention allows for an accurate registration and thus tracking of an optical shape sensing enabled device despite a possible misalignment of the proximal part of the distal optical fiber with the distal part of the proximal optical fiber in the optical connector. While registration methods exist, which allow registration of a FORS enabled device to an imaging system, if there is no connector or if there is a 'perfect' connector in the optical path, these registration methods cannot be simply applied to a FORS system comprising not only the fixed launch base, but also a 'non-perfect' connector in the optical shape sensing path. The present invention is based on the insight that not only the transformation (rotation and translation) from the fixed launch base (which is the starting point of the optical shape measurement up to the tip of the device and thus of the three-dimensional shape reconstruction of the device) to the coordinate system of the imaging system (i.e. to the real world) has to be determined in a registration procedure, but also the transformation in the (not perfect) optical connector between the two fiber parts optically connected with one another is needed to be determined, to correctly localize the device, e.g. a guide wire, in the coordinate system of the imaging system. The transformation in the connector to be determined is needed in order to compensate for misalignments in the optical connector. In an initial registration (e.g. during set-up of the shape sensing system), not only the transformation from the launch base to the coordinate system of the imaging system is unknown, but also the transformation in the optical connector is unknown. In the existing conventional registration procedures, the transformations belonging to the launch base and belonging to the optical connector cannot be determined separately. In other words, only the combined transformation can be determined which however is not sufficient for an accurate localization of the distal device in the relevant coordinate system.

The present invention now overcomes this disadvantage by positioning the optical connector in at least two different positions and/or orientations, e.g. with respect to an object, into which the distal optical fiber may be inserted at least in part, and in each position and/or orientation of the optical connector the shape and orientation of the distal optical fiber and the shape of the section of the proximal fiber extending from the optical connector to the launch base is optically measured. That is, during the localization/registration procedure of the distal optical fiber with respect to the imaging system, the position and/or orientation of the optical connector is changed at least one time, e.g. with respect to an object into which the distal optical fiber may be inserted. For at least one of the different positions and/or orientations of the optical connector the distal optical fiber is localized in or registered to the coordinate system of the imaging system on the basis of matching the optically measured shape/orientation with the shape/orientation of the device provided by the data from images of the imaging system. This yields $n \geq 2$ virtual transformations for the fixed launch base. From these $n \geq 2$ virtual transformations, the real transformation of the launch base and that in the optical connector can be then determined. Thus, despite possible misalignments in the optical connector, the registration of the system from the distal tip of the device to and including the launch base to the imaging system can be performed with high accuracy, and thereby the position and orientation of the device, in particular its distal tip, can be accurately tracked during maneuvering the device in an object. The method according to the invention may start once the distal optical fiber is inserted into an object.

A further advantage of the method according to the invention is that no additional images from the imaging system are required.

Registration data obtained by the registration method can then be used in the real-time reconstruction of the device in a procedure or intervention in which the device is used. It is also possible to update the registration data, e.g. continuously, using for example a Kalman Filter or similar technique.

It is to be understood that the steps of the method described herein can be carried out in a different order than described above.

Preferred embodiments of the invention are defined in the dependent claims and are described herein.

At least one of steps ii) and iii) may be performed without moving at least part of the distal optical fiber so that less images, e.g. X-ray images, are needed in the registration process. This may simplify the registration of the distal optical fiber and thus the device comprising the distal optical fiber to the imaging system based on the image data provided by the imaging system.

Further, at least a part of registration data of the registration obtained in step v) may be stored to obtain stored registration data so as to be available after a disconnection and reconnection of the distal optical fiber and the proximal optical fiber at the optical connector. The stored registration data can be advantageously used in a re-registration process after a disconnection and reconnection of the fibers at the optical connector has performed, thus reducing the amount of calculations needed after a reconnection of the fibers at the connector.

It may be further advantageous, if during a disconnection and reconnection of the distal optical fiber and the proximal optical fiber at the optical connector, at least part of the distal optical fiber is not moved during disconnecting the distal optical fiber from the proximal optical fiber. In this embodiment, the shape of the distal optical fiber measured immediately before the disconnection of the fibers at the optical connector may be stored as the registration data and can be advantageously used in a re-registration process after a re-connection of the fibers. Thus, as at least part of the distal optical fiber has a stable position and orientation during disconnect and reconnect, this stable position/orientation replacing X-ray images after reconnect.

The method may further comprise, after re-connecting the distal optical fiber with the proximal optical fiber at the optical connector, providing the stored registration data, optically measuring the shape and orientation of the distal optical fiber, optically measuring the shape and orientation of the proximal optical fiber from the optical connector to the launch base, and registering the optical shape sensing system to the imaging system based on equating the newly measured shape and orientation of the distal optical fiber with the registration data.

In the context with the previous embodiment, the stored registration data may include a shape and orientation of the distal optical fiber optically measured before disconnecting the distal optical fiber from the proximal optical fiber, or the stored registration data may include one or more single points along the distal optical fiber. The one or more points may include a point of entrance of the distal optical fiber into an object or any other single point or points along the distal optical fiber or the device comprising the distal optical fiber.

In another embodiment, the method may comprise, after re-connecting the distal optical fiber and the proximal optical fiber, providing the stored registration data, from a positioning of the optical connector in a third position and/or orientation, optically measuring the shape and orientation of the distal optical fiber and optically measuring the shape and orientation of the proximal optical fiber from the optical connector to the launch base while the optical connector is in the third position and/or orientation, from the positioning of the optical connector in a fourth position and/or orientation, optically measuring the shape and orientation of the distal optical fiber and optically measuring the shape and orientation of the proximal optical fiber from the optical connector to the launch base while the optical connector is in the fourth position and/or orientation, registering the optical shape sensing system to the imaging system based on the optical measurements in the third and fourth positions and/or orientations of the optical connector and the provided registration data. In this embodiment, the registration data preferably is the registration of the launch base to the imaging system before disconnecting the distal optical fiber from the proximal optical fiber. Registering the optical shape sensing system to the imaging system may include registering the part of the distal optical fiber which may be inserted into an object including a distal tip of the distal optical fiber.

According to a second aspect of the present invention, a method of registering part of an optical shape sensing system to an imaging system is provided after a first registration was performed, said part of the optical shape sensing system comprising a distal multicore optical fiber and a proximal multicore optical fiber, an optical connector optically connecting the distal optical fiber and the proximal optical fiber to each other, a launch base fixed with respect to the imaging system and arranged to fix the proximal optical fiber, wherein after the first registration the proximal optical fiber and the distal optical fiber have been disconnected from one another, the method comprising after reconnecting the proximal and the distal optical fiber:

> i) providing stored registration data of the first registration;
> ii) positioning the optical connector in a first position;
>
>> optically measuring the shape and orientation of the distal optical fiber,
>> optically measuring the shape and orientation of a section of the proximal fiber extending from the optical connector to the launch base,
>
> iii) registering said part of the optical shape sensing system as a whole to the imaging system:
>
>> a) based on equating the newly optically measured shape and orientation of the distal optical fiber with the stored registration data, wherein the stored registration data is shape-related or location-related data of the distal optical fiber before disconnecting the proximal optical fiber from the distal optical fiber;
>> or
>> by further comprising positioning the optical connector in a second position and/or orientation, and in the second position and/or orientation, optically measuring the shape and orientation of the distal optical fiber and optically measuring the shape and orientation of a section of the proximal fiber extending from the optical connector to the launch base, based on the optical measurements in the first and second positions and/or orientations of the optical connector and the stored registration data, wherein the stored registration data is data of the first registration of the launch base.

The method according to the second aspect of (re-) registering the optical shape sensing system to the imaging system may be based on stored registration data, which have been obtained in a registration process different from that according to the first aspect. The method according to the second aspect may be performed after a first registration of the optical shape sensing system to the imaging system has been made, wherein the first registration in turn may have been done via stored registration data stored in the optical shape sensing system. The method according to the second aspect, including alternatives a) or b), is or are considered as an invention on its/their own, i.e. independent of the method according to the first aspect. It is to be understood, that the second aspect may have the same or similar embodiments and/or advantages as the method according to the first aspect.

In a third aspect of the present invention, there is provided a system, comprising a distal multicore optical fiber and a proximal multicore optical fiber, an optical connector optically connecting the distal optical fiber and the proximal optical fiber to each other, a launch base fixed with respect to the imaging system and arranged to fix the proximal fiber, and circuitry configured to:

i) provide data of at least a portion of the distal optical fiber from images of the imaging system;
ii) after positioning the optical connector in a first position and/or orientation:

- optically measure the shape and orientation of the distal optical fiber,
- optically measure the shape and orientation of a section of the proximal fiber extending from the optical connector to the launch base,

iii) after positioning the optical connector in a second position and/or orientation different from the first position and/or orientation:

- optically measure the shape and orientation of the distal optical fiber,
- optically measure the shape and orientation of said section of the proximal fiber,

iv) register at least said portion of the distal fiber to the imaging system based on a shape-to-shape basis between at least one of the optical measurements in the first and second positions and/or orientations of the optical connector and the provided data,
v) based on the optical measurements in the first and second positions and/or orientations of the optical connector, register the system as a whole, including at the optical connector and at the launch base, to the imaging system.

In a fourth aspect, a computer program is provided which comprises program code means for causing a computer to perform the steps of the method disclosed herein when said computer program is carried out on a computer, as well as a non-transitory computer-readable recording medium that stores therein a computer program product, which, when executed by a processor, causes the method disclosed herein to be performed.
It shall be understood that the claimed method, system, and computer program have similar and/or identical preferred embodiments, in particular as defined in the dependent claims and as disclosed herein.

BRIEF DESCRIPTION OF THE DRAWINGS

These and other aspects of the invention will be apparent from and elucidated with reference to the embodiment(s) described hereinafter. In the following drawings:

Fig. 1 shows a schematic block diagram of a system for tracking a device;
Fig. 2 shows a schematic drawing of a shape of a device and its projections onto two images of an imaging system;
Fig. 3 shows a part of the system in Fig. 1;
Fig. 4 shows a scheme of transformations from a tip of a device to the imaging system coordinate system;
Fig. 5 shows a transformation in an optical connector connecting two optical fibers;
Fig. 6 shows a step in a method of registering an optical shape sensing system to an imaging system;
Fig. 7 shows a flow diagram of a method of registering an optical shape sensing system to an imaging system;
Fig. 8 shows a further flow diagram of a method of registering an optical shape sensing system to an imaging system;
Fig. 9 shows a further flow diagram of a method of registering an optical shape sensing system to an imaging system;
Fig. 10 shows a representation of a rotation for subsequent rotations around the x, y, z coordinate axes;
Fig. 11 shows a representation of a rotation for a rotation around a rotation vector; and
Fig. 12 shows a representation of yaw, pitch and roll rotations in an optical connector.

DETAILED DESCRIPTION OF THE INVENTION

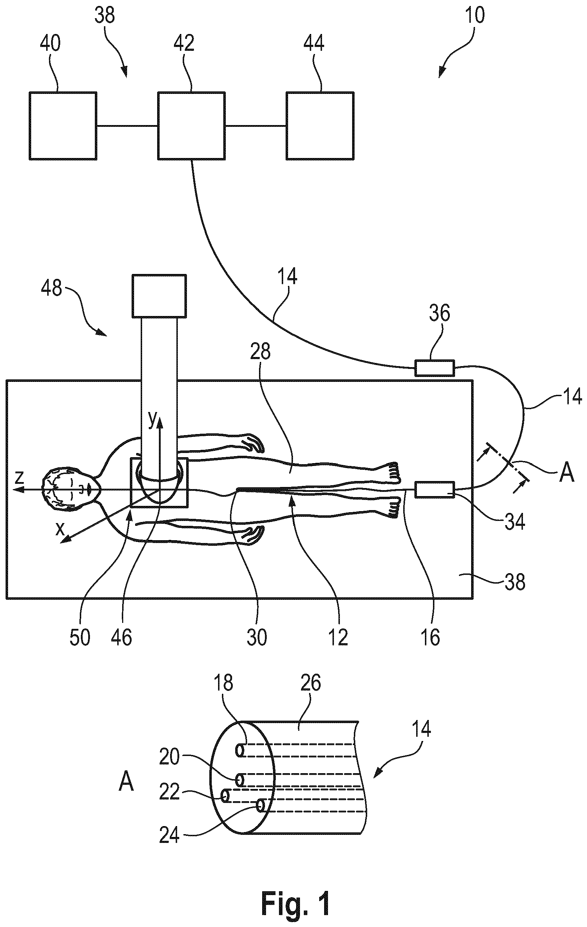

Fig. 1 schematically shows an optical shape sensing system 10 configured to track shape and orientation of an optical shape sensing enabled device 12. Fig. 1 shows the system 10 in a medical procedure. It is to be understood that the present technology is not limited thereto. The system 10 may also be referred to as FORS system.
Optical shape sensing system 10 comprises a proximal multicore optical fiber 14 and a distal multicore optical fiber 16. Proximal multicore optical fiber 14 may be comprised by a patch cord. Distal multicore optical fiber 16 may be comprised by the device 12. Insert A in Fig. 1 shows an example of a portion of a multicore optical fiber in an enlarged cross section. As such, the multicore optical fibers 14 and 16 comprise multiple cores, e.g. cores 18, 20, 22, 24 embedded in a cladding 26. The multiple cores 18, 20, 22, 24 may comprise one central core 20 surrounded by three outer cores 18, 22 and 24. It shall be understood that the number of cores is not limited to 4, but can also be 2, 3 or more than 4. The outer cores 18, 22, 24 may be spiraled around the center core 20. The outer cores may be angularly spaced with respect to one another around the longitudinal center axis of the optical fiber 14 or 16. The center core 20 may be positioned on the longitudinal center axis, with some tolerances due to the fiber manufacturing process. According to a number of four cores with three outer cores in the present example, the angular spacing between neighboring outer cores typically is 120°, again with some tolerances due to the fiber manufacturing process. In particular, the angular and radial positions of the cores 18, 20, 22, 24 of the distal optical fiber 16 may not be exactly the same as the angular and radial positions of the corresponding cores of the proximal optical fiber 14, due to manufacturing tolerances.

The distal multicore optical fiber 16 may be integrated into the device 12. Device 12 may be, for example, a guide wire. Device 12 is shown partially inserted into an object 28. Object 28 is, for example, a body of a patient. A point of entrance or insertion of the device 12 and, thus, the distal multicore optical fiber 16 into the object 28 is denoted with reference numeral 30 in Fig. 1. The proximal optical fiber 14 and the distal optical fiber 16 are connected with one another via an optical connector 34. The proximal optical fiber 14 and the distal optical fiber 16 may be disconnected from one another at the optical connector 34, and can be reconnected again at the optical connector 34. The optical connector 34 forms part of the optical shape sensing system 10. Optical connector 34 connects a distal end of the proximal optical fiber 14 with the proximal end of the distal optical fiber 16. The optical shape sensing system 10 further comprises a launch fixture or launch base 36. The launch base 36 is fixed or stationary in space. In particular, launch base 36 may be fixed with respect to the object 28, and may be fixed to a table 38 on which the object 28 rests. The optical connector 34 is not fixed in space, but is movable with respect to the object 28. Launch fixture 36 is arranged to fix a portion of the proximal optical fiber 14 in space. The proximal multicore optical fiber 14 may pass through the launch base 36 and may be connected to an optical shape sensing console 38. The optical shape sensing console 38 may comprise a light source 40, e.g. a tunable light source which can be swept through a range of optical frequencies. The optical shape sensing console 38 may further comprise an optical interrogation module 42. The optical shape sensing system 10 may be especially configured as a multi-channel optical frequency domain reflectometry-based and distributed-strain sensing system. The interrogation module 42 may be configured for interrogating the optical fibers 14 and 16, more precisely the fiber cores 18, 20, 22, 24 of the optical fibers 14 and 16. The shape sensing console 38 may further comprise a shape reconstruction module 44 arranged to evaluate the light signals reflected or scattered back from the optical fibers 14 and 16 and to reconstruct the shape and orientation of the optical fibers 14 and 16 from the reflected or back-scattered optical signals, wherein reconstruction starts at the launch base 36 and extends up to a distal tip of the device 12, more precisely of the distal optical fiber 16. The reconstruction module 44 may also be arranged for displaying the reconstructed shape and orientation of the device 12, e.g. on a display.

When the light source 22 is swept through a range of optical frequencies (or wavelengths), each fiber core 18, 20, 22 and 24 of the optical fibers 14 and 16 is simultaneously but independently optically interrogated, and the resulting interference pattern from reflected light from each of the fiber cores 18, 20, 22, 24 and reference light is evaluated by the shape reconstruction module 44. The distributed strain measurements are used for 3-dimensional shape reconstruction in particular of the distal optical fiber 16 and thus the device 12 and for visual display of the reconstructed 3-dimensional shape and orientation of the device 12.

In OSS or FORS, geometrical changes of the optical fibers 14 and 16 are encoded into the light field that propagates through the optical fibers. Optical interrogation of the distal optical fiber 16 gives the information needed to, in principle, reconstruct the 3-dimensional shape of the device 12 comprising the fiber 16, in real time. A more detailed overview about the principles of optical shape sensing can be taken from US 2012/0069347 A1 and US 8,773,650 B2.

Given an appropriate coordinate system or reference frame, it is possible to know the exact orientation and position of the device 12 in real time. In order to know the exact orientation and position of the device 12 with respect to an image provided by an imaging system, it is necessary to register the optical shape sensing system 10 from the launch base 36 up to the tip 46 of the distal optical fiber 16, thus the tip 46 of the device 12, to the imaging system. Such an imaging system is shown in Fig. 1 with reference numeral 48. The imaging system 48 may be an X-ray imaging system or any other imaging system, for example a magnetic resonance (MR) imaging system. Fig. 1 illustrates a coordinate system 50 with x, y, and z-axes. In order to reproduce and display the shape and orientation of the distal optical fiber 16 and thus the device 12 correctly overlaid with an image provided by the imaging system 48, it is necessary to register the optical shape sensing system 10 from the launch base 36 up to the tip 46 of the device 12 accurately to the relevant coordinate system 50 defined by the imaging system 48. Shape reconstruction of the optical fibers 14 and 16 up to the tip 46 starts at the launch base 36 so that also the launch base 36 must be correctly registered to the imaging system 48.

Registration methods exist which work well if there is no connector like connector 34 between the launch base 36 and the tip 46 of the optical fiber 16. When the optical connector 34 is present to make a functional connection between the distal optical fiber 16 and the proximal optical fiber 14, it is important to align the fiber cores 18, 20, 22, 24 of the distal optical fiber 16 with the corresponding cores 18, 20, 22 and 24 of the proximal optical fiber 14. A common way to achieve such an alignment is to provide an optical connector with low tolerance elements in the connector, such as ceramic ferrules, to ensure the centering and clocking of the two fibers inside the mating sleeve. Proper clocking is achieved by a connector key which defines the angular alignment between the outer cores of the two optical fibers. However, for certain designs of medical devices 12, for example guide wires, in particular back loadable guide wires, where the outer diameter typically cannot be larger than the fraction of a millimeter, a robust connector key is difficult to manufacture. This holds even more for so-called connectors with backloading capability. In some surgical procedures, a catheter around the optical guide wire needs to be replaced by another one to carry out a medical treatment of the patient. In those procedures, the optical guide wire stays inside the patient, while the catheter around the guide wire is replaced by another one. Thus, a back loadable optical connector 34 is needed, which can disconnect the distal optical fiber from the proximal optical fiber to interchange the catheter. After exchanging the catheter, the distal and proximal optical fibers 14 and 16 are reconnected in the optical connector 34. Due to non-zero tolerances in the fabrication process of an optical fiber like the optical fiber 14 and/or the optical fiber 16 and in that of the optical connector 34, it is most likely that a misalignment between the fiber cores 18, 20, 22, 24 of the distal optical fiber 16 and the proximal optical fiber 14 occur. These non-zero tolerances, and hence misalignments, have a detrimental effect on the accuracy of the predicted position and orientation of the device 12, in particular near its distal tip 46.

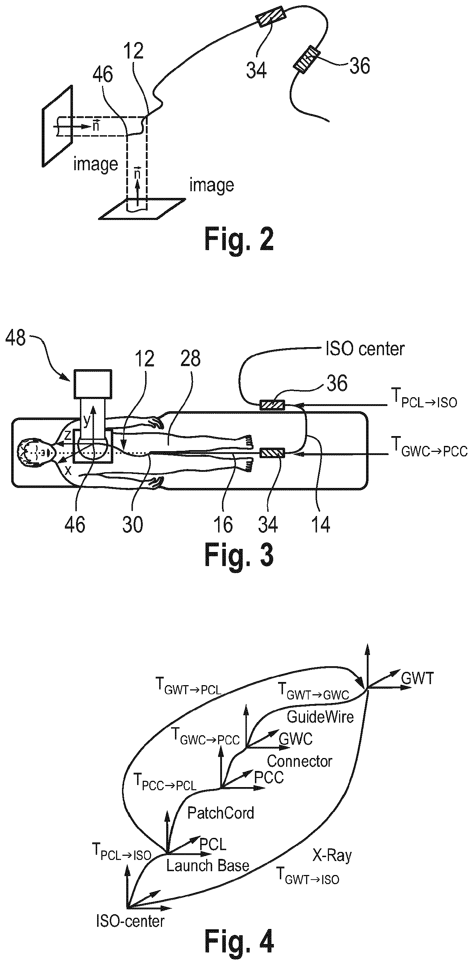

As mentioned above, methods exist to register a device like the device 12 to the relevant coordinate system linked to an imaging system if there is no optical connector 34 or if there is a perfect optical connector in the optical path from the launch base 36 to the distal tip 46 of the optical fiber 16. In these registration methods, the mathematical transformation is determined which is needed to match the optically determined shape of the device 12 to that in e.g. two images of the device 12, e.g. two X-ray images, made under different angles. Fig. 2 schematically shows an example shape of the device 12 near its distal tip. The device 12 can be localized with one shape transformation such that the projections of this shape onto the planes of the images are matched with the shapes of the device 12 in the images. In Fig. 2, $\vec{n}$ denotes the normal vector on the image planes.

If there is the optical connector 34 in the optical path from the launch base 36 to the tip 46 of the device 12, not only the transformation belonging to the launch base 36 is unknown, but also the transformation in the optical connector 34. With the existing registration methods, these two unknown transformations cannot be determined separately. Only the combined transformation can be determined.

Fig. 3 shows the drawing of Fig. 1 again, with the transformations $T_{PCL \rightarrow ISO}$ from the launch base 36, to which the proximal optical fiber 14 is fixed, to the ISO center, and the transformation $T_{GWC \rightarrow PCC}$ from the proximal optical fiber 14 to the distal optical fiber 16 in the connector 34. The ISO center is fixedly linked with the imaging system 48, i.e. the relevant coordinate system to which the device 12 is to be registered.

Fig. 4 shows an overview of the transformations in the device 12 path from the ISO center to the tip 46 of the device 12. The transformation $T_{GWT \rightarrow ISO} = T_{PCL \rightarrow ISO} \, T_{GWT \rightarrow PCL}$ from the ISO center to the device tip 46 (GWT) can be determined with the existing registration method described above with respect to Fig. 2.

Optical interrogation of the optical fiber 16 provides measured signals from which the shape and orientation of the device 12 with respect to the launch base 36 are determined. Therefore, the transformation $T_{GWT \rightarrow PCL}$ from the device tip 46 to the launch base 36 is known from these measured signals. Then, the static transformation $T_{PCL \rightarrow ISO}$ from the launch base 36 to the ISO center follows from

$$T_{PCL \rightarrow ISO} = T_{GWT \rightarrow ISO} \, T^{-1}_{GWT \rightarrow PCL}.$$

Hence, for all subsequent transient motions of the device 12 (outside and inside the object 28), the shape and orientation of the device 12, especially near the distal tip 46, follow from optically interrogating the optical fiber 16 and processing the measured signals:

$$T_{GWT \rightarrow ISO} (t) = T_{PCL \rightarrow ISO} \, T_{GWT \rightarrow PCL} (t)$$

The transformation $T_{PCL \rightarrow ISO}$ is static during movements of the device 12, as the proximal fiber 14 is fixed to the launch base 36.

When an optical connector like optical connector 34, in particular a back loadable connector, is inserted in the optical fibers 14, 16 between the launch base 36 and the point of insertion 30 of the optical fiber 16 into the object 28 (here the patient), an extra unknown transformation $T_{GWC \rightarrow PCC}$ in the optical connector 34 needs to be determined to accurately determine the shape and orientation of the device 12 inside the object 28.

Thus, the transformation $T_{GWT \rightarrow ISO}$, when the optical connector 34 is taken into account, can be written as:

$$T_{GWT \rightarrow ISO} = T_{PCL \rightarrow ISO} \, T_{PCC \rightarrow PCL} \, T_{GWC \rightarrow PCC} \, T_{GWT \rightarrow GWC} \qquad (1)$$

Without limiting the generality, the abbreviations GWT, PCC, GWC, GWT refer to a case where the device 12 is a guide wire (GW) and the optical fiber 14 is arranged in a patch cord (PC), with the following meanings:

PCL = patch cord at launch base 36
PCC = patch cord at optical connector 34
GWC = guide wire at optical connector 34
GWT = guide wire tip 46

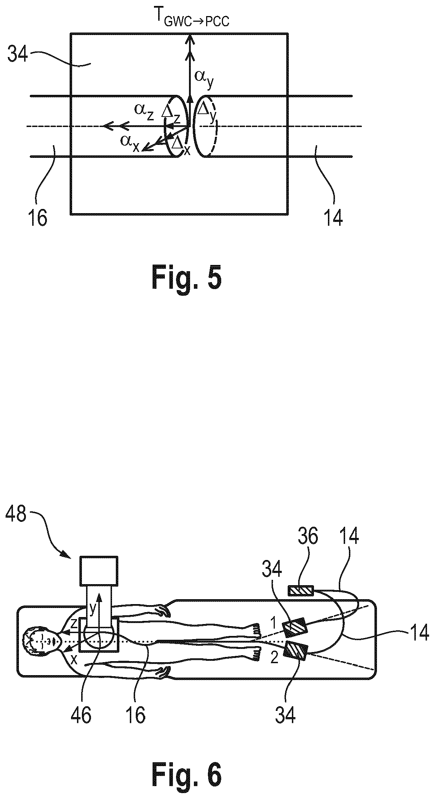

The optical determination of the shape and orientation of the optical fibers 14 and 16 by the optical shape sensing system 10 starts at the launch base 36. The shape and orientation of the portion of the proximal optical fiber 14 (e.g. within the patch cord) which extends from the connector 34 to the launch base 36, is determined from the optically measured signals obtained by optically interrogating the proximal optical fiber 14. The connector 34 connects the distal optical fiber 16 (e.g. within the guide wire or device 12) to the proximal optical fiber 14 (e.g. within the patch cord). The shape and orientation of the distal optical fiber 16 from the connector 34 to the distal tip 46 of the optical fiber 16 (e.g. within the guide wire or device 12) is also determined from the optically measured signals obtained by interrogating the distal optical fiber 16. In the connector 34, the distal end of the proximal optical fiber 14 and the proximal end of the distal optical fiber 16 are connected, as shown in Fig. 5, by a yet unknown transformation $T_{GWC \rightarrow PCC}$. Hence, the two unknown transformations that need to be determined are the transformation $T_{PCL \rightarrow ISO}$ from the launch base 36 to the ISO center and the transformation $T_{GWC \rightarrow PCC}$ in the connector 34, in particular if the connector 34 is a connector, e.g. a back loadable connector, where a misalignment between the proximal optical fiber and the distal optical fiber 16 is likely to occur.

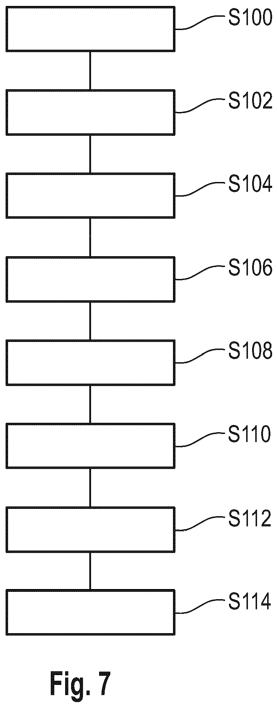

In the following, an embodiment of a method of registering part of the optical shape sensing system 10, which part comprises the distal multicore optical fiber 16 and the proximal multicore optical fiber 14, the optical connector 34 optically connecting the distal optical fiber 16 and the proximal optical fiber 14 to each other, and the launch base 36 fixed with respect to the imaging system 48 and arranged to fix the proximal optical fiber 14 will be described. The method enables determination of the two unknown transformations $T_{PCL \rightarrow ISO}$ and $T_{GWC \rightarrow PCC}$. The embodiment of the method will be described with reference to Fig. 7.

In a step S100, data of at least a portion of the distal optical fiber 16 from images of the imaging system 48 are provided. If the imaging system 48 is an X-ray imaging system, the images are X-ray images of the distal optical fiber 16, especially in the region of the distal tip 46 of the optical fiber 16.

In a step S102, the optical connector 34 is positioned in a first position or orientation with respect to the object 28. The first position or orientation may be arbitrary. For example, the optical connector 34 may be positioned in position or orientation 1 as shown in Fig. 6. In the first position or orientation 1 of the optical connector 34, a step S104 is performed. In step S104, the shape and orientation of the distal optical fiber 16 and the shape and orientation of a section of the proximal optical fiber 14 extending from the optical connector 34 to the launch base 36 are optically measured. That is, the interrogation module 42 optically interrogates the optical fibers 14 and 16, and the shape reconstruction module 44 reconstructs the 3-dimensional shape and orientation of the optical fibers 14 and 16 up to the tip 46 of the distal optical fiber 16 from the back-scattered or back-reflected optical signals received from the fibers 14, 16.

In a step S106, the optical connector 34 is positioned or oriented in a second position or orientation different from the first position or orientation 1 with respect to the object 28. The second position or orientation is arbitrary, wherein it is only necessary that the second position or orientation is (sufficient) different from the first position or orientation 1. In Fig. 6, the second position or orientation of the optical connector 34 is denoted with 2.

In the second position or orientation 2 of the optical connector 34, a step S108 is performed. In step S108, the shape and orientation of the distal optical fiber 16 and the shape and orientation of the section of the proximal optical fiber 14 from the optical connector 34 to the launch base 36 are optically measured again. This optical measurement of the shape and orientation of the distal optical fiber 16 and of said section of the proximal optical fiber 14 are performed like in the first position or orientation of the optical connector 34.

In a step S110, at least the portion of the distal fiber 16 for which images of the imaging system are provided, is registered to the imaging system 48 according to the existing registering method, i.e. based on a shape-to-shape basis between at least one of the optical shape measurements in the first or second positions or orientations of the optical connector 34 and the provided images. In particular, this registration step may be performed for each of the positions or orientations of the optical connector 34 and the corresponding optical shape measurements in these positions.

It is to be noted that step S110 may be performed on the basis of the (X-ray) image data obtained in step S100. Nevertheless, the image may be taken in step S110, or in step S108 in arbitrary order with performing the optical shape measurement. Further, for example, step S110 may be performed after S104 and before step S106, using the optical shape measurements of the distal and proximal optical fibers 14, 16 in the first position or orientation of the optical connector 34. Also, step S110 may be performed after step S108 after the optical shape measurement of the distal and proximal optical fiber 14, 16 in the second position or orientation of the optical connector 34, however without requiring a further image of the device 12. Another possible sequence of steps may be starting with step S102, followed by steps S100 and S104 afterwards in random order, then step S106 can be performed. In step S108, a (X-ray) image may be taken in arbitrary order with the optical shape measurement, or the image may be taken in step S100 or S110.

It is further to be understood that steps S104 and S108 may be performed for more than two different positions or orientations of the connector 34 with respect to the object 28. Thus, for n ≥ 2 different positions or orientations of the optical connector 34, n virtual transformations $T^i_{GWT \to ISO}$, i = 1, ... n are obtained. Next, the real transformation $T_{PCL \to ISO}$ belonging to the launch base 36 and the real transformation $T_{GWC \to PCC}$ belonging to the optical connector 34 can be determined from the set of n equations:

$$T^i_{GWT \to ISO} = T_{PCL \to ISO} \; T^i_{PCC \to PCL} \; T_{GWC \to PCC} \; T^i_{GWT \to GWC}, \; i = 1, ..., n \quad (2)$$

The transformations $T^i_{PCC \to PCL}$ and $T^i_{GWT \to GWC}$ have been obtained from the optically measured and reconstructed shapes and orientations of the optical fiber 16 and the proximal optical fiber 14, for each position or orientation i = 1, ..., n of the optical connector 34. More precisely, the transformations $T^i_{PCC \to PCL}$ are obtained from the optically measured shapes and orientations of the optical fiber 14 from the connector 34 to the launch base 36, and the transformations $T^i_{GWT \to GWC}$ are obtained from the optically measured shapes and orientations of the distal optical fiber 16 from the distal tip 46 to the connector 34. The transformation $T^i_{GWT \to ISO}$ is obtained from the localization of at least the distal part of the distal optical fiber 16 in the images of the imaging system (e.g. as illustrated in Fig. 2). Thus, in case of n = 2, the unknown transformations $T_{PCL \to ISO}$ and $T_{GWC \to PCC}$ can be determined from solving the two (i=1 and i=2) equations (2) above for these two unknown transformations. It can be convenient to rewrite these equations as:

$$T^{-1}_{PCL \to ISO} \; T^i_{GWT \to ISO} = T^i_{PCC \to PCL} \; T_{GWC \to PCC} \; T^i_{GWT \to GWC}, \; i = 1, ..., n \quad (3)$$

so that the unknown transformations:

$T^{-1}_{PCL \to ISO}$ and $T_{GWC \to PCC}$
must be solved. Once $T^{-1}_{PCL \to ISO}$ and $T_{GWC \to PCC}$ are known, also the transformation $T_{PCL \to ISO}$ is easily determined.

Thus, in step S112 in Fig. 7, registration data are computed to register the part of the optical shape sensing system from the launch base 36 to the tip of the distal fiber 16 including at the connector 34 and the launch base 36 to the imaging system 48, based on the optical shape measurements in the first and second positions or orientations of the optical connector 34.

Further, the method may further comprise a step S114 in which at least the part of the registration data obtained in step S112 is stored, for example in a memory of the optical shape sensing system 10.

After registration of the optical shape sensing system as described above, the device 12 can be accurately tracked and visualized in the correct position and orientation with respect to the relevant coordinate system of the imaging system 48.

In some surgical procedures, a catheter around the optical guide wire needs to be replaced by another one to carry out a medical treatment of the patient. In such procedures, the patch cord with the proximal optical fiber 14 is to be disconnected from the distal optical fiber 16 at the optical connector 34. While the device 12 with the distal optical fiber 16 stays inside the object 28 (patient) the catheter around the device 12 is replaced by another one. To this end, the optical connector 34 may be a back loadable optical connector.

If the distal optical fiber 16 is disconnected and afterwards reconnected in the optical connector 34 with the proximal optical fiber 14, the transformation $T_{GWC \to PCC}$ belonging to the optical connector 34 between the two connecting parts of the proximal optical fiber 14 and the distal optical fiber 16 has or may have changed. This changed transformation needs to be determined again to correctly localize the device 12 inside the body for further treatments of the patient.

In other words, the optical shape sensing system 10 from the launch base 36 to the tip 46 of the device 12 has to be re-registered after reconnect of the distal and proximal optical fibers 16 and 14.

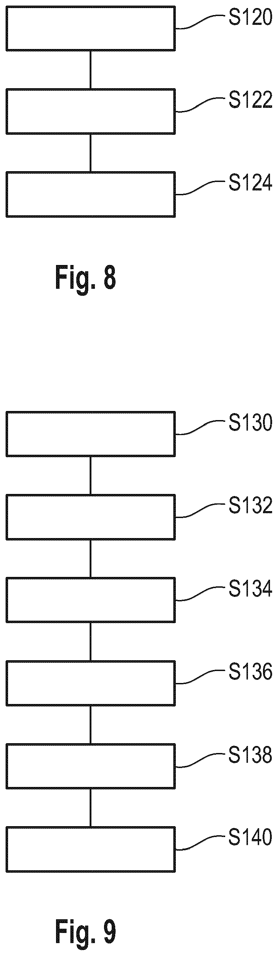

In a first embodiment of the re-registration method according to Fig. 8, registration data of the registration before the proximal and the distal optical fibers 14 and 16 have been disconnected, and which have been stored as stored registration data, are provided in a step S120. The stored registration data may include a shape and orientation of the distal optical fiber 16 optically measured before disconnecting the distal optical fiber from the proximal optical fiber, or the stored registration data may include one or more single points along the distal optical fiber 16, especially the point of entrance 30 of the distal optical fiber 16 into the object 28. In the present embodiment, it is essential that the part of the distal optical fiber 16 which is inserted into the object 28 is not moved during disconnecting the distal optical fiber 16 from the proximal optical fiber 14 and reconnecting same.

Thus, the shape and orientation of the distal optical fiber 16 before disconnecting the optical connector 34 is known. Hence, the transformations belonging to the launch base 36 ($T_{PCL \to ISO}$) and that belonging to the optical connector 34 (before disconnect) $T_{GWC \to PCC} = T^{before}_{GWC \to PCC}$ are known. After reconnecting the distal optical fiber 16 in the optical connector 34 with the proximal optical fiber 14, the shapes and orientations of the parts of the proximal optical fiber from the connector 34 to the launch base 36 and from the tip 46 of the distal optical fiber 16 to the connector 34 are optically measured and reconstructed in a step S122.

If the stored registration data include the shape and orientation of the device 12 and thus the distal optical fiber 16 before disconnect, the registration of the optical shape sensing system 10 from the launch base 36 to the tip 46 of the distal optical fiber 16 to the imaging system 48 can be performed by equating the newly measured shape and orientation of the distal optical fiber 16 with the stored registration data in a step S 124. If the stored registration data include the point of entrance 30 of the distal optical fiber 16 into the object 28 (or if the stored registration data includes other points along the device 12), the point of entrance 30 is newly determined (or other points along the device 12 are newly determined), and registration is performed by equating this or these newly determined point(s) with the stored point(s) in step S124.

In terms of transformations to be determined, the method of re-registering described before can be expressed as follows: As the shape and orientation of the device 12 before disconnecting the optical connector 34 is known, the transformation belonging to the launch base 36 ($T_{PCL \to ISO}$) and that belonging to the optical connector 34 (before disconnect) $T^{before}_{GWC \to PCC}$ are known. After reconnecting the distal optical fiber 16 in the optical connector 34 with the proximal optical fiber 14, the shapes and orientations of the parts of the optical fiber 14 from the connector 34 to the launch base 36 and of the optical fiber 16 from the tip 46 to the connector 34 are optically measured and reconstructed. Hence, the transformations $T^{after}_{PCC \to PCL}$ and $T^{after}_{GWT \to GWC}$ after disconnecting and reconnecting the optical connector 34 are determined and the unknown changed transformation $T^{after}_{GWC \to PCC}$ needs to be determined. As the location of the distal optical fiber 16 inside the object 28 was known before disconnecting the distal optical fiber 16 and it was not moved, it is still at the same location inside the object 28 after reconnecting. Therefore, the total transformation $T_{GWT \to ISO}$ before and the total transformation after the disconnect-reconnect procedure are the same:

$$T^{before}_{GWT \to ISO} = T^{after}_{GWT \to ISO} = T.$$

This yields:

$$T_{PCL \to ISO} \; T^{before}_{PCC \to PCL} \; T^{before}_{GWC \to PCC} \; T^{before}_{GWT \to GWC} = T = T_{PCL \to ISO} \; T^{after}_{PCC \to PCL}$$

$$T^{after}_{GWC \to PCC} \; T^{after}_{GWT \to GWC}, \hspace{2cm} (4)$$

or analogously

$$T^{before}_{PCC \to PCL} \; T^{before}_{GWC \to PCC} \; T^{before}_{GWT \to GWC} = T^{-1}_{PCL \to ISO} \; T = T^{after}_{PCC \to PCL} \; T^{after}_{GWC \to PCC}$$

$$T^{after}_{GWT \to GWC} \hspace{3cm} (5)$$

The transformation $T^{after}_{GWC \to PCC}$ at the connector 34 can now be determined from equation (5) above.

Fig. 9 shows another embodiment of a method of (re-)registering part of the optical shape sensing system 10 from the launch base 36 to the tip of the distal optical fiber 16 to the imaging system 48.

Some steps of this embodiment are similar to the steps of the method according to Fig. 7. Differently from the method according to Fig. 7, now the position and orientation of the launch base 36 is known and due to small movements of the device 12 and thus the distal optical fiber 16 inside the object 28, the position and orientation of the distal tip 46 of the distal optical fiber 16 is considered to be unknown. Hence, considering unknowns, the distal tip 46 of the distal optical fiber 16 and the launch base 36, for which the transformation in the embodiment of Fig. 7 was unknown, are now interchanged. In other words, the position and orientation of the fiber 14 at the launch base 36 is known before disconnecting the distal optical fiber 16 from the connector 34 and the launch base 36 is fixed with respect to the real world during the disconnect-reconnect procedure of the distal optical fiber 16. Thus, the method according to Fig. 9 starts with providing in a step S130 stored registration data of the registration before the disconnect, wherein the registration data is the registration of the launch base 36 to the imaging system 48 before disconnecting the distal optical fiber 16 from the proximal optical fiber 14, wherein step S130 is performed after the reconnect of the distal and the proximal optical fibers 16, 14.

Then, in a step S132, the optical connector 34 is positioned in a first position or orientation.

In a step S134, the shape and orientation of the distal optical fiber 16 and the shape and orientation of the proximal optical fiber 14 from the optical connector 34 to the launch base 36 are optically measured as described above.

Then, in a step S136, the optical connector 34 is positioned in a second position or orientation, and in a step S138 the shape and orientation of the distal optical fiber 16 and of the proximal optical fiber from the optical connector 34 to the launch base 36 is optically measured again. It is to be noted that the second position or orientation is different from the first position or orientation of the optical connector 34. As in the method according to Fig. 7, the optical connector 34 may be positioned in more than two different positions or orientations, and the optical measurements are performed in each of these different positions.

In a step S140, the optical shape sensing system 10 from the launch base 36 to the tip 46 of the distal optical fiber 16 is registered to the imaging system 48 based on the optical measurements in the first and second positions or orientations of the optical connector 34 and the registration data, i.e. the registration of the launch base 36 to the imaging system 48 before disconnect of the distal optical fiber 16 from the proximal optical fiber 14.

It is to be noted that the position and orientation of the tip of the distal optical fiber 16 is fixed between the two positions or orientations of the optical connector 34.

In terms of transformations, the embodiment described before may be explained as follows:

For $m \geq 2$ different orientations or positions of the connector 34 with respect to the object 28, the shapes and orientations of the proximal optical fiber 14 from the optical connector 34 to the launch base 36 and of the distal optical fiber 16 are optically measured and reconstructed. The transformations $T^i_{PCC \to PCL}$, $i = 1, ..., m$, are obtained from the optically measured shapes and orientations of the part of the optical fiber 14 from the connector 34 to the launch base 36 and the transformations $T^i_{GWT \to GWC}$, $i = 1, ..., m$, are obtained from the optically measured shapes and orientations of the part of the distal optical fiber 16 from the distal tip 46 to the connector 34. The unknown transformation $T_{GWT \to ISO}$ belonging to the distal tip 46, describing the position and orientation of the device tip 46 with respect to the fixed world, and the transformation $T_{GWC \to PCC}$ belonging to the optical connector 34 are determined from the following set of equations:

$$T_{GWT \to ISO} = T_{PCL \to ISO} \; T^i_{PCC \to PCL} \; T_{GWC \to PCC} \; T^i_{GWT \to GWC}, \; i = 1, ..., m, \quad (6)$$

wherein $T_{PCL \to ISO}$ is the known transformation belonging to the launch base 36 obtained from the stored registration data of a previous, e.g. a first, registration.

It is to be noted that the embodiments according to Fig. 8 and according to Fig. 9 do not require that the method according to Fig. 7 has been performed previously. The embodiments of (re-) registering the optical shape sensing system 10 to the imaging system 48 may be based on stored registration data, which have been obtained by a (previous or first) registration process different from that in Fig. 7. That is, the embodiments according to Fig. 8 and Fig. 9 may be performed after a first or previous registration of the optical shape sensing system 10 to the imaging system 48 has been made, wherein the first or previous registration in turn may have been done via stored registration data stored in the optical shape sensing system 10.

List of reference signs in Fig. 7 to 9:

| S100 | Providing data from images from imaging system |
| S102 | Positioning optical connector in first position/orientation |
| S104 | Optically measuring distal/proximal optical fiber |
| S106 | Positioning optical connector in second position and/or orientation |
| S108 | Optically measuring distal/proximal optical fiber |
| S110 | Registering distal optical fiber to imaging system based on images |
| S112 | Registering optical shape system based on optical measurements in first and second positions/orientations of optical connector |
| S114 | Storing registration data |

| S120 | Providing stored registration data |
| S122 | Optically measuring distal/proximal optical fiber |
| S124 | Equating optical measurement with registration data |

| S130 | Providing registration data of registration before disconnect |
| S132 | Positioning optical connector in first position/orientation |
| S134 | Optically measuring distal/proximal optical fiber |
| S136 | Positioning optical connector in second position and/or orientation |

S138    Optically measuring distal/proximal optical fiber
S140    Registering optical shape sensing system based on optical measurements in first and second positions of optical connector and registration data before disconnect

*Description of Solution of the equations according to Fig. 7, 8 or 9*

A transformation $T$ consists of a rotation, with rotation matrix $R$, and a translation $\vec{p}$ and is written as

$$T = \begin{bmatrix} R & \vec{p} \\ \vec{0}^T & 1 \end{bmatrix}$$ . Then, the inverse transformation reads $$T^{-1} = \begin{bmatrix} R^T & -R^T\vec{p} \\ \vec{0}^T & 1 \end{bmatrix}$$ , as rotation matrices have the property $RR^T = R^TR = I$. The transformation $T$ operates on vectors $\vec{y}$ that consist of the coordinates $\vec{x}$ of a point in the 3D-space and the additional number 1, hence $T$ operates on vectors $\vec{y} = (\vec{x}^T, 1)^T$ and the transformation yields

$$T\vec{y} = \begin{bmatrix} R & \vec{p} \\ \vec{0}^T & 1 \end{bmatrix} \begin{pmatrix} \vec{x} \\ 1 \end{pmatrix} = \begin{pmatrix} R\vec{x} + \vec{p} \\ 1 \end{pmatrix}$$ . Note, the first 3 elements of $T\vec{y}$ describe the transformation of the point $\vec{x}$ and the 4$^{th}$ element is the number 1, similar to the vector $\vec{y}$.

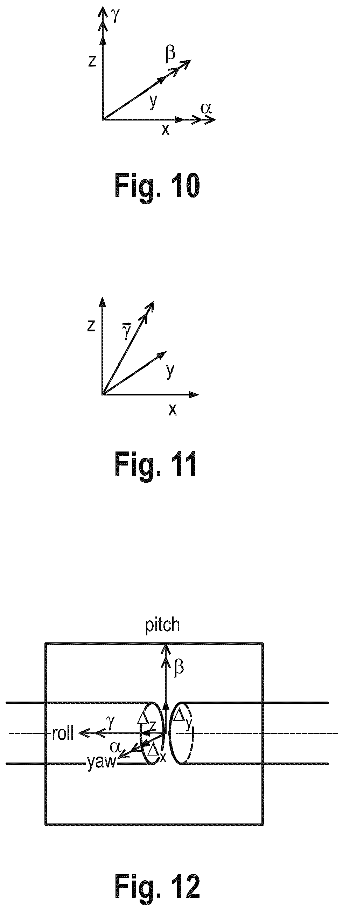

The rotation matrix R can be written as the subsequent rotations around the three orthogonal $x$, $y$ and $z$ coordinate axes, (see e.g. Fig. 10), hence R $= R_z(\gamma)R_y(\beta)R_x(\alpha)$, where $R_i$, $i = x, y, z$, indicates the rotation around the coordinate axis i with the Euler angle $\alpha, \beta, \gamma$, respectively. The rotation matrices $R_i$ are

$$R_x(\alpha) = \begin{bmatrix} 1 & 0 & 0 \\ 0 & \cos\alpha & -\sin\alpha \\ 0 & \sin\alpha & \cos\alpha \end{bmatrix}, R_y(\beta) = \begin{bmatrix} \cos\beta & 0 & \sin\beta \\ 0 & 1 & 0 \\ -\sin\beta & 0 & \cos\beta \end{bmatrix}, R_z(\gamma) =$$

$$\begin{bmatrix} \cos\gamma & -\sin\gamma & 0 \\ \sin\gamma & \cos\gamma & 0 \\ 0 & 0 & 1 \end{bmatrix} .$$

(7)

Alternatively, (see e.g. Fig. 11), the rotation matrix R can be derived from a rotation vector $\vec{r} = \theta\vec{k}$. which represents the rotation around the unit vector $\vec{k}$ with angle $\theta$. Then, using the formula of DeRodrigues, the rotation matrix reads

$$R = I + \sin\theta K + (1 - \cos\theta)K^2 ,$$

(8)

$$K = \begin{bmatrix} 0 & -k_z & k_y \\ k_z & 0 & -k_x \\ -k_y & k_x & 0 \end{bmatrix}$$
with $\vec{k} = (k_x, k_y, k_z)^T$ and $|\vec{k}| = 1$.

*Equations belonging to the embodiments according to Fig. 7 to 9*

Using the representation of a transformation above, the n $\geq$ 2 equations to determine the solution of the embodiment according to Fig. 7 are rewritten as

$$\begin{bmatrix} R_{GWT\to ISO} & \vec{p}_{GWT\to ISO} \\ \vec{0}^T & 1 \end{bmatrix}_i = \begin{bmatrix} R_L & \vec{p}_L \\ \vec{0}^T & 1 \end{bmatrix} \begin{bmatrix} R_{C\to L} & \vec{p}_{P\to L} \\ \vec{0}^T & 1 \end{bmatrix}_i \begin{bmatrix} R_C & \vec{p}_C \\ \vec{0}^T & 1 \end{bmatrix} \begin{bmatrix} R_{T\to C} & \vec{p}_{T\to C} \\ \vec{0}^T & 1 \end{bmatrix}_i , \quad i =$$

$$1 \cdots n ,$$

(9)

from which the unknown rotation matrices $R_L$ and $R_C$ and transformations $\vec{p}_L$ and $\vec{p}_C$ belonging to the launch base 36 and to the (back loadable) optical connector 34, respectively, need to be solved. Analogously the simplified equations can be rewritten as

$$\begin{bmatrix} Q_L & \vec{q}_L \\ \vec{0}^T & 1 \end{bmatrix} \begin{bmatrix} R_{GWT \to ISO} & \vec{p}_{GWT \to ISO} \\ \vec{0}^T & 1 \end{bmatrix}_i = \begin{bmatrix} R_{C \to L} & \vec{p}_{P \to L} \\ \vec{0}^T & 1 \end{bmatrix}_i \begin{bmatrix} R_C & \vec{p}_C \\ \vec{0}^T & 1 \end{bmatrix} \begin{bmatrix} R_{T \to C} & \vec{p}_{T \to C} \\ \vec{0}^T & 1 \end{bmatrix}_i, \; i = \quad (10)$$

$$1 \cdots n \,,$$

from which the unknown rotation matrices $Q_L$ and $R_C$ and transformations $\vec{q}_L$ and $\vec{p}_C$ belonging to the launch base 36 and to the connector 34, respectively, need to be solved. Thereafter, the rotation matrix $R_L$ and translation $\vec{p}_L$ of the launch base 36 are easily found as $R_L = Q_L^T$ and $\vec{p}_L = -Q_L^T \vec{q}_L$.

Likewise, in the embodiment of Fig. 8 or 9, the transformation belonging to the connector 34 after a disconnect-reconnect procedure can be rewritten as

$$\begin{bmatrix} R_{C \to L}^{before} & \vec{p}_{C \to L}^{before} \\ \vec{0}^T & 1 \end{bmatrix} \begin{bmatrix} R_C^{before} & \vec{p}_C^{before} \\ \vec{0}^T & 1 \end{bmatrix} \begin{bmatrix} R_{T \to C}^{before} & \vec{p}_{T \to C}^{before} \\ \vec{0}^T & 1 \end{bmatrix} =$$

$$\begin{bmatrix} R_{C \to L}^{after} & \vec{p}_{C \to L}^{after} \\ \vec{0}^T & 1 \end{bmatrix} \begin{bmatrix} R_C^{after} & \vec{p}_C^{after} \\ \vec{0}^T & 1 \end{bmatrix} \begin{bmatrix} R_{T \to C}^{after} & \vec{p}_{T \to C}^{after} \\ \vec{0}^T & 1 \end{bmatrix}. \quad (11)$$

The unknown changed rotation matrix $R_C^{after}$ and changed transformation $\vec{p}_C^{after}$ after a disconnect-reconnect procedure in the connector 34 need to be solved.

The equations above can form an overdetermined set of equations, hence in that case the number of independent equations is larger than the number of unknowns, due to inaccuracies in the determination of the shapes of the proximal fiber 14 (patch cord) and of the distal fiber 16 (guide wire). These inaccuracies can be caused by (small) inaccuracies in the measured signals and by (small) inaccuracies (discretization errors) in determining the shapes and orientations of the patch cord and of the guide wire from the measured signals. The set of equations belonging to the embodiment of Fig. 7 or 9 can be overdetermined especially if n > 2. Analogously, the set of equations belonging to the embodiment of Fig. 8 can be overdetermined if multiple reference points on the guide wire are selected before a disconnect and these points of the guide wire are matched onto the original reference points after reconnecting the guide wire. In the case of an overdetermined set of equations belonging to all embodiments of Fig. 7, 8 or 9, the set of equations can be solved in a least squares sense. Analytical, semi-analytical and/or numerical methods can be applied to solve the (overdetermined) set of equations derived above belonging to the embodiments of Fig. 7, 8 or 9. The solution is the set of two unknown transformations $T_{PCL \to ISO}$ and $T_{GWC \to PCC}$ for the registration of the optical device or it is the unknown changed transformation $T_{GWC \to PCC}^{after}$ (after disconnect-reconnect).

It must be ensured that the rotation matrix R in the transformations T above satisfies the orthogonality condition $RR^T = R^T R = I$. The total set of equations for the unknowns will be non-linear, therefore most probably an iterative procedure is needed to solve them. As the rotations and the translations in the connector 34 are expected to be small, an initial estimate for the rotation matrix R in the connector 34 can be the identity matrix $I$ and an initial estimate for the translation $\vec{p}$ in the connector can be the zero translation $\vec{0}$.

In the case that the variation between the different orientations/positions of the connector 34, in the solution for the embodiment of Fig. 7, is very small, the obtained set of equations may become ill-conditioned and it will be difficult or impossible to determine a proper unique solution. In that case the condition number of the gradient matrix (derivatives of the equations with respect to the unknowns) will be large, see https://en.wikipedia.org/wiki/Condition_number (a problem with a low condition number is said to be well-conditioned, while a problem with a high condition number is said to be ill-conditioned) or https://nl.mathworks.com/help/symbolic/cond.html. If the condition number of the set of equations is large, a notification should be given to the operator to enlarge the variation in the orientations and/or positions of the connector 34.

In the case of an overdetermined set of equations, alternatively the condition number of the least squares set of equations can be used instead of that of the original set of equations. Suppose that the unknowns are represented in the vector $\vec{z}$ and the set of equations is denoted by $\vec{F}(\vec{z}) = \vec{0}$, then the least squares solution satisfies

$$\left(\vec{\nabla}\vec{F}\right)^{T}\vec{F}(\vec{z}\,) = \left(\vec{\nabla}\vec{F}\right)^{T}\vec{0} = \vec{0}\,, \tag{12}$$

and the condition number from the matrix $(\vec{\nabla}\vec{F})^{T}\vec{\nabla}\vec{F}$ can be used, instead of that of $\vec{\nabla}\vec{F}$, to notify the operator if the set of equations is well-conditioned or ill-conditioned and if he/she should enlarge the variation in the orientations and/or positions of the connector bloc. Note, this matrix is used in the application of a Newton iterative procedure to solve the set of overdetermined non-linear equations in a least squares sense, as the Newton iterative procedure reads

$$\left(\vec{\nabla}\vec{F}(\vec{z}^{k}\,)\right)^{T}\vec{\nabla}\vec{F}(\vec{z}^{k}\,)\,\Delta\vec{z}^{k} = -\left(\vec{\nabla}\vec{F}(\vec{z}^{k}\,)\right)^{T}\vec{F}(\vec{z}^{k}\,)\,,\ \ \vec{z}^{k+1} = \vec{z}^{k} + \Delta\vec{z}^{k}\,. \tag{13}$$

If the distal fiber 16 (guide wire) is disconnected from the connector 34, the part of the guide wire inside the patient should be kept stable. A notification should be given to the operator to ensure that the part of the guide wire inside the patient should be kept stable.

*Reduction of the unknowns in the transformation belonging to the optical connector*

The unknown roll rotation in the connector due to the non-zero tolerances in the manufacturing processes is expected to have the largest effect on the inaccuracy of the predicted position of the distal tip area of the device 12. The effect on the position of the distal tip 46 of the device 12 of the translation and the yaw and pitch rotations around the other two axes in the connector are expected to be negligibly small, see Fig. 12. Therefore, the unknown transformation in the connector 34 most probably can be simplified to only an unknown roll motion with the roll angle y as the only unknown variable. In that case the transformation $T_{GWC \to PCC}$ may be approximated by

$$T_{GWC \to PCC} \approx \begin{bmatrix} \cos\gamma & -\sin\gamma & 0 & \\ \sin\gamma & \cos\gamma & 0 & \vec{0} \\ 0 & 0 & 1 & \\ & \vec{0}^{T} & & 1 \end{bmatrix}, \tag{1}$$

where the local z-axis is in the length direction of the guide wire.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Method of registering part of an optical shape sensing system (10) to an imaging system (48), said part comprising a distal multicore optical fiber (16) and a proximal multicore optical fiber (14), an optical connector (34) optically connecting the distal optical fiber (16) and the proximal optical fiber (14) to each other, a launch base (36) fixed with respect to the imaging system (48) and arranged to fix the proximal optical fiber (14), the method comprising:

   i) providing data of at least a portion of the distal optical fiber (16) from images from the imaging system (48);
   ii) from the positioning of the optical connector (34) in a first position and/or orientation, and in the first position and/or orientation of the optical connector (34):

- optically measuring the shape and orientation of the distal optical fiber (16),
- optically measuring the shape and orientation of a section of the proximal fiber (14) extending from the optical connector (34) to the launch base (36),

iii) from the positioning of the optical connector (34) in a second position and/or orientation different from the first position and/or orientation, and in the second position and/or orientation of the optical connector (34):

- optically measuring the shape and orientation of the distal optical fiber (16),
- optically measuring the shape and orientation of said section of the proximal fiber (14),

iv) registering at least said portion of the distal fiber (16) to the imaging system based on a shape-to-shape basis between at least one of the optical shape measurements in the first and second positions and/or orientations of the optical connector (34) and the provided data,
v) based on the optical measurements in the first and second positions and/or orientations of the optical connector (34), registering said part of the optical shape system as a whole, including at the optical connector (34) and at the launch base (36), to the imaging system (48).

2. Method of claim 1, wherein at least one of steps ii) and iii) is performed without moving at least part of the distal optical fiber (16).

3. Method of claim 1, further comprising storing at least a part of registration data of the registration obtained in step v) to obtain stored registration data so as to be available after a disconnection and reconnection of the distal optical fiber (16) and the proximal optical fiber (14) at the optical connector (34).

4. Method of claim 3, wherein, during a disconnection and reconnection of the distal optical fiber (16) and the proximal optical fiber (14) at the optical connector (34), at least part of the distal optical fiber (16) is not moved during disconnecting the distal optical fiber (16) from the proximal optical fiber (14).

5. Method of claim 3, further comprising, after re-connecting the distal optical fiber (16) with the proximal optical fiber (14) at the optical connector (34), providing the stored registration data, optically measuring the shape and orientation of the distal optical fiber (16), optically measuring the shape and orientation of the proximal optical fiber (14) from the optical connector (34) to the launch base (36), and registering the optical shape sensing system (10) to the imaging system (48) based on equating the newly measured shape and orientation of the distal optical fiber (16) with the registration data.

6. Method of claim 5, wherein the stored registration data includes a shape and orientation of the distal optical fiber (16) optically measured before disconnecting the distal optical fiber (16) from the proximal optical fiber (14).

7. Method of claim 5, wherein the stored registration data includes one or more single points along the distal optical fiber (16).

8. Method of claim 7, wherein the one or more points include a point (30) of entrance of the distal optical fiber (16) into an object (28).

9. Method of claim 3, further comprising, after re-connecting the distal optical fiber (16) and the proximal optical fiber (14), providing the stored registration data, from a positioning of the optical connector (34) in a third position and/or orientation, optically measuring the shape and orientation of the distal optical fiber (16) and optically measuring the shape and orientation of the proximal optical fiber (14) from the optical connector (34) to the launch base (36) while the optical connector (34) is in the third position and/or orientation, from the positioning of the optical connector (34) in a fourth position and/or orientation, optically measuring the shape and orientation of the distal optical fiber (16) and optically measuring the shape and orientation of the proximal optical fiber (14) from the optical connector (34) to the launch base (36) while the optical connector (34) is in the fourth position and/or orientation, registering the optical shape sensing system (10) to the imaging system (48) based on the optical measurements in the third and fourth position and/or orientation of the optical connector (34) and the provided registration data.

10. Method of claim 9, wherein the registration data is the registration of the launch base (36) to the imaging system (48) before disconnecting the distal optical fiber (16) from the proximal optical fiber (14).

**11.** Method of claim 9, wherein registering the optical shape sensing system (10) to the imaging system (48) includes registering at least part of the distal optical fiber (16) including a distal tip (46) of the distal optical fiber (16).

**12.** System, comprising a distal multicore optical fiber (16) and a proximal multicore optical fiber (14), an optical connector (34) optically connecting the distal optical fiber (16) and the proximal optical fiber (14) to each other, a launch base (36) fixed with respect to the imaging system (48) and arranged to fix the proximal fiber (14), and circuitry configured to:

i) provide data of at least a portion of the distal optical fiber (16) from images from the imaging system (48);
ii) after positioning the optical connector (34) in a first position and/or orientation:

- optically measure the shape and orientation of the distal optical fiber (16),
- optically measure the shape and orientation of a section of the proximal fiber (14) extending from the optical connector (34) to the launch base (36),

iii) after positioning the optical connector (34) in a second position and/or orientation different from the first position or orientation:

- optically measure the shape and orientation of the distal optical fiber (16),
- optically measure the shape and orientation of said section of the proximal fiber (14),

iv) register at least said portion of the distal fiber (16) to the imaging system (48) based on a shape-to-shape basis between at least one of the optical measurements in the first and second positions or orientations of the optical connector (34) and the provided data,
v) based on the optical measurements in the first and second positions or orientations of the optical connector (34), register the system (10) as a whole, including at the optical connector (34) and at the launch base (36), to the imaging system (48).

**13.** Computer program comprising program code means for causing a computer to carry out the method as claimed in claim 1, when said computer program is carried out on said computer.

Fig. 1

Fig. 2

Fig. 3

Fig. 4

**Fig. 5**

**Fig. 6**

S100

S102

S104

S106

S108

S110

S112

S114

Fig. 7

S120

S122

S124

Fig. 8

S130

S132

S134

S136

S138

S140

Fig. 9

Fig. 10

Fig. 11

Fig. 12

Europäisches
Patentamt

European
Patent Office

Office européen
des brevets

# EUROPEAN SEARCH REPORT

Application Number

EP 22 17 4889

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 995 076 A1 (KONINKLIJKE PHILIPS NV [NL]) 11 May 2022 (2022-05-11) | 12 | INV.<br>A61B34/20 |
| A | * figures 1-11 *<br>* paragraph [0046] – paragraph [0092] * | 1-11,13 | |
| Y | US 2014/155737 A1 (MANZKE ROBERT [DE] ET AL) 5 June 2014 (2014-06-05) | 12 | |
| A | * paragraph [0003] – paragraph [0004] *<br>* paragraph [0030] – paragraph [0031] *<br>* paragraph [0032] * | 1-11,13 | |
| Y | US 2018/256131 A1 (BRACKEN JOHN ALLAN [US] ET AL) 13 September 2018 (2018-09-13) | 12 | |
| A | * paragraph [0002] *<br>* paragraph [0033] – paragraph [0035] *<br>* paragraph [0044] – paragraph [0045] * | 1-11,13 | |

**TECHNICAL FIELDS
SEARCHED        (IPC)**

A61B
G02B

The present search report has been drawn up for all claims

| Place of search | Date of completion of the search | Examiner |
|---|---|---|
| Munich | 25 October 2022 | Lüddemann, Tobias |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.......................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 17 4889

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2022

| Patent document cited in search report | | Publication date | Patent family member(s) | | Publication date |
|---|---|---|---|---|---|
| EP  3995076 | A1 | 11-05-2022 | EP       3995076 | A1 | 11-05-2022 |
| | | | WO    2022096345 | A1 | 12-05-2022 |
| US 2014155737 | A1 | 05-06-2014 | BR 112014003281 | A2 | 18-04-2017 |
| | | | CN      103781418 | A | 07-05-2014 |
| | | | EP       2744407 | A1 | 25-06-2014 |
| | | | JP       6114748 | B2 | 12-04-2017 |
| | | | JP    2014526927 | A | 09-10-2014 |
| | | | RU    2014110007 | A | 27-09-2015 |
| | | | US    2014155737 | A1 | 05-06-2014 |
| | | | WO    2013024418 | A1 | 21-02-2013 |
| US 2018256131 | A1 | 13-09-2018 | US    2018256131 | A1 | 13-09-2018 |
| | | | WO    2017051279 | A1 | 30-03-2017 |

EPO FORM P0459

For more details about this annex : see Official Journal of the European Patent Office, No. 12/82

**EP 4 282 366 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120069347 A1
- US 8773650 B2